Europäisches Patentamt

European Patent Office

Office européen des brevets

(19)

(11) Numéro de publication : **0 062 952**
**B1**

(12) **FASCICULE DE BREVET EUROPÉEN**

(45) Date de publication du fascicule du brevet :
07.08.85

(51) Int. Cl.⁴ : **C 07 C 19/05, C 07 C 17/42**

(21) Numéro de dépôt : **82200418.0**

(22) Date de dépôt : **02.04.82**

(54) **Compositions stabilisées de 1,1,1-trichloroéthane.**

(30) Priorité : **13.04.81 FR 8107528**

(43) Date de publication de la demande :
**20.10.82 Bulletin 82/42**

(45) Mention de la délivrance du brevet :
**07.08.85 Bulletin 85/32**

(84) Etats contractants désignés :
**AT BE CH DE FR GB IT LI LU NL SE**

(56) Documents cités :
**FR-A- 1 284 254**
**FR-A- 2 105 205**
**FR-A- 2 108 016**
**FR-A- 2 178 898**
**US-A- 3 957 893**
**Le dossier contient des informations techniques présentées postérieurement au dépôt de la demande et ne figurant pas dans le présent fascicule.**

(73) Titulaire : **SOLVAY & Cie (Société Anonyme)**
**Rue du Prince Albert, 33**
**B-1050 Bruxelles (BE)**

(72) Inventeur : **Servais, Michel**
**Chaussée de Malines, 315**
**B-1950 Kraainem (BE)**
Inventeur : **Crochet, Roger**
**Chaussée de Roodebeek 526**
**B-1200 Bruxelles (BE)**

Jouve, 18, rue St-Denis, 75001 Paris, France

**0 062 952**

**Description**

La présente invention concerne des compositions stabilisées de 1,1,1-trichloroéthane, utilisables notamment pour le dégraissage des métaux et pour le nettoyage à sec des textiles.

On sait que le 1,1,1-trichloroéthane, qui est utilisé notamment pour le dégraissage des métaux, le nettoyage à sec des textiles, et dans les aérosols, pose un problème de stabilité et de corrosion tout particulier. L'emploi, pour stabiliser le 1,1,1-trichloroéthane, des stabilisants habituellement utilisés pour d'autres hydrocarbures chlorés ne donne pas, en général, des résultats satisfaisants. Ce phénomène a été attribué notamment au fait qu'il existe une grande réactivité entre le 1,1,1-trichloroéthane et les métaux, tels que l'aluminium, le zinc et leurs alliages, et l'eau. Dès lors, on a développé des compositions stabilisées, spécifiques du 1,1,1-trichloroéthane, nombreuses et variées. parmi celles-ci on peut citer notamment des compositions ternaires à base d'époxydes, de nitroalcanes et de dioxanne décrites dans le document US-A-3 049 571 déposé le 18.3.1960 par THE DOW CHEMICAL CO. Toutefois, aucune des compositions proposées jusqu'à ce jour n'est vraiment efficace.

Par ailleurs on a également déjà proposé dans les documents FR-A-2 108 016 et FR-A-2 105 205 des compositions destinées à stabiliser le 1,1,1-trichloroéthane en vue d'éviter sa décomposition et la formation simultanée d'acide corrosif au cours des stockages et des utilisations. Ces documents divulguent respectivement des compositions à base de 2-méthyl-3-butyne-2-ol et de un ou plusieurs stabilisants choisis parmi les 2-méthyl-3-butène-2-ol, l'alcool amylique tertiaire, le trioxane, l'époxybutane, la méthyléthylcétone, le méthylisothiocyanate, le norbornadiène, le 2-méthylfurane, le dioxolane ou le nitrométhane (FR-A-2 108 016) et à base de 2-méthylfurane et un ou plusieurs costabilisants choisis parmi les alcools, les éthers internes, les nitriles, les isothiocyanates ou les cycloalkadiènes (FR-A 2 105 205).

Aucune composition connue de 1,1,1-trichloroéthane ne présente cependant une stabilité satisfaisante dans des conditions extrêmement sévères telles que celles prévalant lors de la présence simultanée de métaux légers et d'eau.

La présente invention vise à remédier à ces inconvénients des compositions connues et à procurer des compositions stabilisées de 1,1,1-trichloroéthane dont la stabilité est satisfaisante même dans les conditions d'utilisation les plus sévères.

L'invention concerne à cet effet des compositions stabilisées de 1,1,1-trichloroéthane contenant un composé époxydé, un dérivé nitré et un éther interne dans lesquelles l'éther interne est un composé furannique.

Par composé époxydé, on entend les composés aliphatiques saturés ou insaturés contenant au moins un groupement époxyde dans leur molécule. De préférence, les compositions selon l'invention contiennent des composés aliphatiques saturés contenant de 3 à 6 atomes de carbone dans leur molécule tels que l'époxypropane, l'époxybutane, les 2-méthylépoxypropanes, les 2-méthylépoxybutanes, le glycidol et l'épichlorhydrine. De bons résultats ont été obtenus avec le 2-méthyl-2,3-époxybutane et l'époxybutane.

La quantité totale de composé époxydé présente dans les compositions selon l'invention varie habituellement entre 0,01 et 50 grammes par litre. De préférence, cette quantité est comprise entre 0,1 et 20 grammes par litre. Tout particulièrement préférées sont des quantités comprises entre 1 et 10 grammes par litre.

Les dérivés nitrés présents dans les compositions selon l'invention sont de préférence des nitroalkanes contenant de 1 à 4 atomes de carbone dans leur molécule. De bons résultats ont été obtenus avec le nitrométhane, le nitroéthane et les nitropropanes 1 ou 2. Tout particulièrement préférés sont le nitroéthane et surtout le nitrométhane.

Les quantités de composés nitrés sont les mêmes que celles définies pour les composés époxydés.

Par composé furannique, on entend désigner le furanne ainsi que les dérivés du furanne où certains atomes d'hydrogène sont substitués par d'autres atomes ou radicaux et plus particulièrement par des radicaux aliphatiques saturés comprenant de 1 à 5 atomes de carbone.

Généralement, dans les dérivés substitués, le noyau furannique n'est substitué que par un ou deux radicaux aliphatiques et les positions de substitution se situent habituellement sur les atomes de carbone 2 et 5 du noyau furannique.

Parmi les composés furanniques préférés figurent le 2-méthylfurane, le 2-éthylfurane, le 2-isopropylfurane, le 2-propylfurane et le 2,5-diméthylfurane. Les meilleurs résultats ont été obtenus avec le 2-méthylfurane qui est tout particulièrement préféré.

Les quantités de composé furannique présentes dans les compositions selon l'invention varient en général entre 0,1 et 100 g par litre. De préférence, cette quantité est comprise entre 1 et 75 g par litre. Tout particulièrement préférées sont des quantités comprises entre 5 et 50 g par litre.

Outre les composés précités, les compositions selon l'invention peuvent contenir un ou plusieurs autres stabilisants habituels du 1,1,1-trichloroéthane tels que des alcools, des esters, des nitriles, des cétones, ainsi que des composés hétérocycliques contenant un cycle oxaziridine dans la molécule.

Les alcools peuvent être saturés ou insaturés. Les alcools préférés contiennent de 1 à 7 atomes de carbone et peuvent être substitués ou non.

2

Habituellement, les propanols, les butanols tertiaire et secondaire et le pentanol tertiaire sont utilisés. Les alcools saturés préférés sont le butanol tertiaire et l'alcool amylique tertiaire.

Les alcools insaturés habituellement utilisés sont des alcools non substitués contenant de 3 à 6 atomes de carbone. Parmi ceux-ci on préfère l'alcool allylique, le 2-méthyl-3-butène-2-ol. et le 2-méthyl-3-butyne-2-ol. L'alcool insaturé préféré est le 2-méthyl-3-butyne-2-ol.

Comme ester, on utilise le plus souvent des mono- et des diesters contenant de 2 à 8 atomes de carbone saturés ou non, pouvant être substitués par des halogènes ou des groupes hydroxyles. L'acétate d'éthyle, l'acétate d'isobutyle, l'acétate de n-propyle, le méthacrylate de méthyle et l'acétate de n-butyle sont employés le plus fréquemment. Le méthacrylate de méthyle, l'acétate d'éthyle et l'acétate de n-propyle se sont révélés particulièrement adéquats.

Les nitriles le plus souvent mis en œuvre sont des composés aliphatiques ou aromatiques, saturés ou non saturés, éventuellement substitués, contenant de 2 à 7 atomes de carbone. Parmi ceux-ci l'acétonitrile, le propionitrile et l'acrylonitrile conviennent le mieux.

Comme cétones, on peut utiliser des cétones contenant de 3 à 7 atomes de carbone, substituées ou non, telles que par exemple l'acétone, la méthyléthylcétone, la diéthylcétone, la méthylpropylcétone, la méthylisopropylcétone, la 1(2 furyl) propanone, la 3-hydroxy-2,5-hexanedione, la 2-méthyl-2-hydroxy-4-pentanone, etc.

Les dérivés hétérocycliques contenant un noyau oxaziridine dans la molécule sont en général choisis parmi la 2-tert-butyloxaziridine, le 2-tert-amyloxaziridine, la 2-isopropyloxaziridine, la 3-méthyl-2-tert-butyloxaziridine, la 3-méthyl-2-tert-amyloxaziridine, la 3-méthyl-2-isopropyloxaziridine, la 3,3-diméthyl-2-tert-butyloxaziridine, la 3,3-diméthyl-2-tert-amyloxaziridine, la 3,3-diméthyl-2-isopropyloxaziridine, la 3-méthyl-3-éthyl-2-tert-butyloxaziridine, la 3-méthyl-3-éthyl-2-tert-amyloxaziridine, la 3-méthyl-3-éthyl-2-isopropyloxaziridine, la 3-éthyl-2-tert-butyloxaziridine, la 3-éthyl-2-tert. amyloxaziridine, le 3-éthyl-2-isopropyloxaziridine, la 3-propyl-2-isopropyloxaziridine et la 3-isopropyl-2-isopropyloxaziridine. Parmi ceux-ci, la 2-tert-butyloxaziridine, la 2-tert-amyloxaziridine ét la 2-isopropyloxaziridine conviennent le mieux.

D'autres composés et notamment les alkoxyalcanes tels que les alkoxyéthanes, les nitrates tels que les nitrates d'alkyle contenant de 1 à 5 atomes de carbone, les amines tels que les amines aliphatiques contenant de 2 à 6 atomes de carbone, les pyrroles tels que le N-méthylpyrrole et d'autres composés aromatiques tels que le phénol et ses dérivés et le toluène ou aliphatiques tels que le diisobutylène peuvent être également incorporés dans la formule de stabilisation du 1,1,1-trichloroéthane mise finalement en œuvre.

Les différents autres stabilisants utilisés sont généralement présents dans des quantités comprises entre 0,01 et 50 g par litre et de préférence dans des quantités comprises entre 0,1 et 30 g par litre.

Les compositions selon l'invention peuvent être utilisées dans toutes les applications requérant une bonne stabilité du 1,1,1-trichloroéthane. Elles peuvent être utilisées dans des conditions sévères et en particulier en présence simultanée d'éléments métalliques notamment les métaux légers et d'eau.

Ainsi, les compositions selon l'invention peuvent être saturées en eau sans que cela affecte leurs performances. En outre, on peut également les utiliser au contact d'une phase aqueuse.

Les compositions de l'invention présentent également une excellente aptitude au recyclage.

## Exemple 1

Cet exemple est effectué afin de montrer la stabilité améliorée observée avec les compositions selon l'invention (essai 1) en comparaison avec d'autres compositions du même type (essais 2 à 7) dans des conditions opératoires sévères c'est-à-dire en présence de métaux légers et d'eau. Les résultats obtenus dans les différents essais ont été rassemblés au Tableau 1 ci-après.

## Essai 1

Dans un Bécher de 100 cm³ on introduit 50 cm³ de 1,1,1-trichloro-éthane, stabilisé au moyen de 4 g/l de 1,2-époxybutane, 5 g/l de nitrométhane et 30 g/l de 2-méthylfuranne, traité préalablement par agitation en présence d'une quantité égale d'eau et récupération du 1,1,1-trichloroéthane ainsi traité, par décantation.

Tout en maintenant le mélange à températaure ambiante on introduit ensuite une éprouvette de 40 × 20 × 2 mm en alliage d'aluminium contenant 5 % de cuivre, 1,6 % de magnésium et 0,7 % de manganèse (alliage AU5G1-T3) ; cette éprouvette est rayée, après immersion dans le mélange, avec une pointe métallique, tout en maintenant l'éprouvette sous le niveau du liquide pendant l'opération.

La corrosion du métal au niveau de la griffe est alors observée au cours du temps ; cette corrosion se traduit par l'apparition de produits brun foncé, accompagnée d'un dégagement gazeux. Pour l'appréciation des résultats, la corrosion est appelée forte lorsqu'elle est suffisante pour provoquer pendant la première heure, le noircissement total du liquide ainsi qu'un dégagement d'acide chlorhydrique. La corrosion est appelée faible lorsqu'elle a lieu mais sans provoquer les phénomènes décrits ci-avant, pendant la première heure. La corrosion est appelée nulle lorsqu'on n'observe pas la moindre attaque du métal pendant au moins 12 heures.

**0 062 952**

Essais 2, 3, 4, 5, 6 et 7

On répète les opérations de l'essai 1 mais en mettant en œuvre du 1,1,1-trichloroéthane stabilisé au moyen de 4 g/l d'époxybutane, 5 g/l de nitrométhane et de respectivement 30 g/l de 1,4-dioxanne (essai 2), 30 g/l d'alcool butylique tertiaire (essai 3), 30 g/l de 1,3,5 trioxanne (essai 4), 30 g/l d'acétonitrile (essai 5), 30 g/l de dioxolanne (essai 6) et 30 g/l de méthyléthylcétone (essai 7).

Tableau 1

| N° de l'essai | Corrosion observée | | |
|---|---|---|---|
| | Forte | Faible | Nulle |
| 1 (selon l'invention) | | | X |
| 2 (comparaison) | | X | |
| 3 (comparaison) | X | | |
| 4 (comparaison) | X | | |
| 5 (comparaison) | X | | |
| 6 (comparaison) | | X | |
| 7 (comparaison) | X | | |

On peut donc conclure de l'examen des résultats du Tableau 1 que la composition selon l'invention (essai 1) est la seule à ne pas provoquer de corrosion après 12 heures dans les conditions sévères de l'essai (en présence simultanée de métaux légers et d'eau). Par contre, aucune des combinaisons connues (essais 2 à 7) ne permet d'éviter une corrosion faible (essais 2 et 6) ou forte (essais 3, 4, 5 et 7) des métaux après déjà 1 heure d'essai.

Exemple 2

Cet exemple est réalisé afin de montrer l'excellente aptitude au recyclage, sans addition de stabilisant frais, des compositions selon l'invention (essai 1) par comparaison avec des compositions connues qui leur sont comparables (essai 2).

Essai 1

On introduit 1 400 cm³ de 1,1,1-trichloroéthane, stabilisé au moyen de 4 g/l de 1,2-époxybutane, 5 g/l de nitrométhane et 30 g/l de 2-méthylfuranne, additionné de 25 % d'huile de paraffine dans un ballon de 2 500 cm³ équipé d'un réfrigérant et raccordé à une source de vapeur.

La vapeur à 110 °C est ensuite introduite dans le fond du ballon et le mélange est distillé par entraînement à la vapeur jusqu'à récupération de 99,3 % de la quantité de 1,1,1-trichloroéthane mis en œuvre.

On laisse décanter le distillat et on récupère 50 cm³ de 1,1,1-trichloroéthane stabilisé sur lesquels on effectue le test décrit à l'exemple 1, essai 1, sans toutefois effectuer de traitement d'agitation en présence d'eau.

Le restant du 1,1,1-trichloréthane stabilisé est ensuite remis dans le ballon de départ et traité, en présence d'huile de paraffine fraîche dans les conditions décrites ci-avant. Cette opération est répétée 20 fois et la corrosion provoquée par les différentes fractions collectées figure au Tableau 2 ci-après.

Essai 2

On opère selon le mode opératoire de l'essai 1 mais en mettant en œuvre du 1,1,1-trichloroéthane stabilisé au moyen de 4 g/l d'époxybutane, 5 g/l de nitrométhane et 30 g/l de 1,4 dioxanne.

Les résultats de corrosion observée sur les fractions collectées sont également rassemblés au Tableau 2.

4

**0 062 952**

Tableau 2

| N° de la distillation | Corrosion observée | |
| --- | --- | --- |
| | Essai 1 (invention) | Essai 2 (de comparaison) |
| 1 | Nulle | Nulle |
| 2 | " | Nulle |
| 3 | " | Faible |
| 4 | " | Faible |
| 5 | " | Forte |
| 6 | " | |
| 7 | " | |
| 8 | " | |
| 9 | " | |
| 10 | " | |
| 11 | " | |
| 12 | " | |
| 13 | " | |
| 14 | " | |
| 15 | " | |
| 16 | " | |
| 17 | " | |
| 18 | " | |
| 19 | " | |
| 20 | " | |

L'examen des résultats du Tableau 2 montre que la composition selon l'invention (essai 1) maintient sa stabilité quel que soit le nombre de recyclages qu'on lui fait subir. La composition de comparaison (essai 2), par contre, voit ses capacités stabilisantes se réduire après deux opérations de recyclage de sorte qu'elle nécessite l'adjonction fréquente de stabilisants frais.

**Revendications**

1. Compositions stabilisées de 1,1,1-trichloroéthane contenant un composé époxydé, un dérivé nitré et un éther interne caractérisées en ce que l'éther interne est un composé furannique.

2. Compositions selon la revendication 1 caractérisées en ce que les composés furanniques sont substitués par un ou deux radicaux aliphatiques saturés comprenant de 1 à 5 atomes de carbone.

3. Compositions selon la revendication 1 ou 2 caractérisées en ce que le composé furannique est le 2-méthylfuranne, le 2-éthylfuranne, le 2-isopropylfuranne, le 2-propylfuranne ou le 2,5 diméthylfuranne.

4. Compositions selon la revendication 3 caractérisées en ce que le composé furannique est le 2-méthylfuranne.

5. Compositions selon l'une quelconque des revendications 1 à 4 caractérisées en ce que les composés furanniques sont présents dans des quantités comprises entre 1 et 75 g par litre.

6. Compositions selon l'une quelconque des revendications 1 à 5 caractérisées en ce que le composé époxydé est le 2-méthyl-2,3 époxybutane ou le 1,2 époxybutane et en ce que le dérivé nitré est le nitrométhane ou le nitroéthane.

7. Compositions selon l'une quelconque des revendications 1 à 6 caractérisées en ce que les composés époxydés et le dérivé nitré sont présents dans des quantités comprises entre 0,1 et 20 g par litre.

8. Compositions selon l'une quelconque des revendications 1 à 7 caractérisées en ce que le composé époxydé est le 1,2-époxybutane, le composé nitré est le nitrométhane et le composé furannique est le 2-méthyl-furanne.

**Claims**

1. Stabilised compositions of 1,1,1-trichloroethane containing an epoxide compound, a nitro

5

derivative and an internal ether, characterised in that the internal ether is a furan compound.

2. Compositions according to claim 1, characterised in that the furan compounds are substituted by one or two saturated aliphatic radicals containing from 1 to 5 carbon atoms.

3. Compositions according to claim 1 or 2, characterised in that the furan compound is 2-methylfuran, 2-ethylfuran, 2-isopropylfuran, 2-propylfuran or 2,5-dimethylfuran.

4. Compositions according to claim 3, characterised in that the furan compound is 2-methylfuran.

5. Compositions according to any one of claims 1 to 4, characterised in that the furan compounds are present in amounts of between 1 and 75 g per litre.

6. Compositions according to any one of claims 1 to 5, characterised in that the epoxide compound is 2-methyl-2,3-epoxybutane or 1,2-epoxybutane and in that the nitro derivative is nitromethane or nitroethane.

7. Compositions according to any one of claims 1 to 6, characterised in that the epoxide compounds and the nitro derivative are present in amounts of between 0.1 and 20 g per litre.

8. Compositions according to any one of claims 1 to 7, characterised in that the epoxide compound is 1,2-epoxybutane, the nitro compound is nitromethane and the furan compound is 2-methyl-furan.


**Patentansprüche**

1. Stabilisierte 1,1,1-Trichlorethanzusammensetzungen enthaltend eine epoxidierte Verbindung, eine Nitroverbindung und einen inneren Äther, dadurch gekennzeichnet, daß der innere Äther eine Furanverbindung ist.

2. Zusammensetzungen gemäß Anspruch 1, dadurch gekennzeichnet, daß die Furanverbindungen durch ein oder zwei aliphatische gesättigte Reste mit 1 bis 5 Kohlenstoffatomen substituiert sind.

3. Zusammensetzungen gemäß Anspruch 1 oder 2, dadurch gekennzeichnet, daß die Furanverbindung das 2-Methyl-furan, das 2-Äthylfuran, das 2-Isopropylfuran, das 2-Propylfuran oder das 2,5-Dimethylfuran ist.

4. Zusammensetzungen gemäß Anspruch 3, dadurch gekennzeichnet, daß die Furanverbindung das 2-Methylfuran ist.

5. Zusammensetzungen gemäß einem der Ansprüche 1 bis 4, dadurch gekennzeichnet, daß die Furanverbindungen anwesend sind in Mengen zwischen 1 und 75 g per Liter.

6. Zusammensetzungen gemäß einem der Ansprüche 1 bis 5, dadurch gekennzeichnet, daß die epoxidierte Verbindung das 2-Methyl-2,3-epoxybutan oder das 1,2-Epoxybutan ist und daß die Nitroverbindung das Nitromethan oder das Nitroethan ist.

7. Zusammensetzungen gemäß einem der Ansprüche 1 bis 6, dadurch gekennzeichnet, daß die epoxidierten Verbindungen und die Nitroverbindung anwesend sind in Mengen zwischen 0,1 und 20 g per Liter.

8. Zusammensetzungen gemäß einem der Ansprüche 1 bis 7, dadurch gekennzeichnet, daß die epoxidierte Verbindung das 1,2-Epoxybutan, die Nitroverbindung das Nitromethan und die Furanverbindung das 2-Methylfuran ist.